Europäisches Patentamt

European Patent Office

Office européen des brevets

(11) Publication number: 0 313 377
A1

# (12) EUROPEAN PATENT APPLICATION

(21) Application number: 88309907.9

(22) Date of filing: 21.10.88

(51) Int. Cl.⁴: **C 12 P 21/00**
C 07 K 3/22
// C12N15/00

(30) Priority: 23.10.87 US 112800

(43) Date of publication of application:
26.04.89 Bulletin 89/17

(84) Designated Contracting States:
AT BE CH DE ES FR GB GR IT LI LU NL SE

(71) Applicant: SMITHKLINE BECKMAN CORPORATION
One Franklin Plaza P O Box 7929
Philadelphia Pennsylvania 19103 (US)

(72) Inventor: **Deen, Keith Charles**
21 Maude Circle
Paoli Pennsylvania 19301 (US)

**Folena-Wasserman, Gail Marie**
9 Brentwood Place
Holland Pennsylvania 18966 (US)

**Inacker, Richard Henry**
713 Heritage Road
Cinnaminson New Jersey 08077 (US)

**Sweet, Raymond Whitney**
108 Edgehill Road
Bala Cynwyd Pennsylvania 19004 (US)

(74) Representative: **Giddings, Peter John, Dr. et al**
Smith Kline & French Laboratories Ltd. Corporate
Patents Mundells
Welwyn Garden City Hertfordshire AL7 1EY (GB)

(54) Process for purification of soluble T4.

(57) A process for purification of soluble T4 (T4s) from culture
media using gel chromatography techniques is described.

EP 0 313 377 A1

Bundesdruckerei Berlin

Description

## PROCESS FOR PURIFICATION OF SOLUBLE T4

### Field of the Invention

This invention relates to production of a soluble T4 (sT4) protein. More particularly it relates to the construction of vectors for the expression of sT4 in mammalian cells and purification of the resulting product.

### Background of the Invention

The T4 molecule is one of several non-polymorphic T lymphocyte surface proteins that have been implicated in the mediation of efficient T cell-target cell interactions. Analysis of these surface proteins indicates that mature T lymphocytes segregate into two classes on the basis of their predominant expression of either the T4 or TS surface glycoprotein (Reinherz, et al., Cell 19:821(1980)). The T4 molecule is predominantly expressed on helper T lymphocytes whereas T8 is expressed on cytotoxic and suppressor T cells (Reinherz, et al., supra, (1980 ). This functional distinction is not absolute since some T4+ T lymphocytes can effect cytotoxicity and suppression (Thomas, et al., J. Exp. Med. 154:459(1981); Meuer, et al., Proc. Natl. Acad. Sci. USA 79:4395(1982)). A stronger relationship exists between T cell subsets and the major histocompatibility complex (MHC) gene products expressed by the target cells. T4+ T lymphocytes interact with target cells expressing MHC class II gene products whereas T8+ T cells interact with targets expressing class I MHC molecules (Engleman, et al., J. Immunol. 127:2124(1981); Krensky, et al., Proc. Natl. Acad. Sci. USA 79:2365(1982); Biddison, et al., J. Exp. Med. 156:1065(1982); Swain, et al., Immunol. Res. 74:129(1983)). Moreover, monoclonal antibodies directed against T4 and T8 inhibit T cell function in vitro (Engleman, et al., J. Exp. Med. 154:193(1981); Wilde, et al., J. Immunol. 131:2178(1983). These observations suggest that the specificity of interaction of subpopulations of T lymphocytes with different target cells results from the association of T4 and T8 with class II MHC and class I MHC proteins, respectively, and that this recognition may be essential for T cell function.

The cDNA sequence of the human T4 receptor is known (Maddon, et al., Cell 42:93(1985)). The complete T4 pre-protein sequence is 458 amino acids in length comprising the putative 23 amino acid secretory leader, 372 amino acid surface ($V_1V_4$), 23 amino acid transmembrane and 40 amino acid cytoplasmic domains. The surface domain shows four regions of limited homology, 20-30%, to immunoglobulin variable (V) and joining (J) regions. Four of the five intron-exon boundaries in the surface domain occur near the junctions of these V-J regions. On the basis of partial protein sequence information for the mouse and sheep T4 proteins (Classon, et al., Immunogen. 23:129(1986), the 6 cysteines in the surface domain are paired sequentially to give three disulfide bonds. There are two possible sites for N-linked glycosylation based on amino acid sequence. This V-J homology, cysteine pairing and intron-exon structure suggest that T4 shares some structural similarities with the immunoglobulins.

The human immunodeficiency virus (HIV), the etiological agent of acquired immune deficiency syndrome (AIDS), shows a marked affinity for T4+ lymphocytes. Molecular characterization of the HIV genome has demonstrated that the virus exhibits the same overall gag-pol-env organization as other retroviruses. Ratner, et al., Nature 313:277(1985); Wain-Hobson, et al., Cell 40:9(1985). The T4+ lymphotropic character of the virus can be explained by its specific binding to the T4 receptor. Monoclonal antibodies directed against T4 block HIV infection of T4+ cells in vitro (Dalgleish, et al., Nature 312:763(1984); McDougal, et al., J. Immunol. 135:3151(1985)). Human cell lines, nonlymphoid as well as lymphoid, which lack the T4 receptor cannot be infected by HIV, but these cells become susceptible to infection upon introduction and expression of the cloned T4 gene (Maddon, et al., Cell 47:333(1986)). Virus binding to T4 is mediated by the gp120 glycoprotein of the viral envelope. The T4 protein can be co-precipitated from lysed HIV infected cells with anti-env antibody and, conversely, gp120 can be co-precipitated with anti-T4 monoclonal antibody, thus demonstrating that virus binding to T4 is mediated by the gp120 glycoprotein of the viral envelope (McDougal, et al., Science 231:382(1986)).

AIDS results in the impairment, and ultimately the depletion, of T4+ lymphocytes with consequent dysfunction of the cellular immune response. In vitro, HIV specifically kills T4+ cells (Gallo, et al., Science 224:497(1984); Sarngadharan, et al., Science 224:506(1984); Barre-Sinoussi, et al., Science 220:868(1983); however, some lymphoid and myeloid cells can be productively infected without notable cytopathic effect (DeRossi, et al., Proc. Natl. Acad. Sci. USA 83:4297(1986); Gartner, et al., Science 233:215(1986). In vitro, cytotoxicity is dependent on the level of expression of the HIV envelope proteins (Sodroski, et al., Nature 322:470(1986) and the T4 receptor (Hoxie, et al., Science 234:1123(1986). A recent report describes a direct correlation between cytolysis and syncytium formation (Yoffe, et al., Proc. Natl. Acad. Sci. USA 84:1429(1987). However, the cause of cytolysis is not clear; it may be an immediate consequence of cell fusion or the result of the co-expression of HIV env proteins and T4 within the same cell.

The AIDS virus may be neurotropic as well as lymphotropic. AIDS is frequently accompanied by central nervous system (CNS) dysfunction, most often the consequence of a subacute encephalitis. AIDS virus RNA and DNA have been identified in affected brains (Shaw, et al., Science 227:177(1985)); virus has been located within macrophages and astrocytes and virus has been isolated from both brain and cerebrospinal fluid of patients with neurological disorders (Levy, et al., Lancet II:586(1985)). These observations suggest that the AIDS virus infects cells in the brain and is directly responsible for the CNS lesions observed in AIDS patients.

Recent studies have identified the expression of T4 mRNA in some brain tissues (Maddon, et al., supra, (1986). Two RNA species are observed, one comparable in size to that seen in lymphocytes and a second shorter mRNA. The nature of the T4 protein encoded by this shortened mRNA is not yet clear. The relevance of T4 expression in brain tissue to HIV infiltration of the CNS is only speculative at present.

### Summary of the Invention

This invention relates to a process for purifying soluble T4 (sT4) from clarified conditioned medium from a cell culture which produces sT4. The process comprises contacting the conditioned medium with a cation exchange support whereby the sT4 is adsorbed to the support; eluting the sT4 from the support; contacting the eluate with an anion exchange support; and collecting sT4 in the effluent from the anion exchange support.

In another embodiment of the invention a process for producing sT4 is described which comprises the following steps: 1) culturing mammalian cells which express sT4; 2) clarifying the conditioned medium from the cultured cells by centrifugation and filtration; 3) contacting the clarified medium with a sulfopropyl-Sepharose® column; 4) eluting sT4 from the column using a linear gradient of 0-0.5 M NaCl; 5) dialyzing the eluate to remove the salt; 6) contacting the dialyzed eluate with a QAE-Sepharose® column; 7) passing the effluent through a column of Superose® 12; and 8) collecting the purified sT4.

These and other embodiments are considered further aspects of the same invention which is fully disclosed herein.

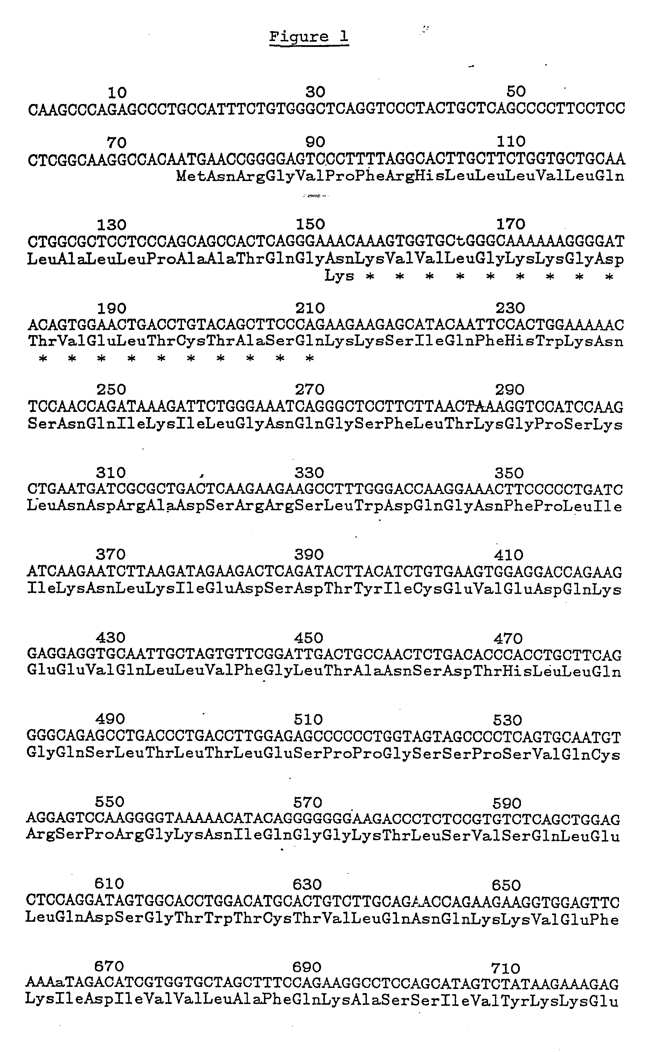

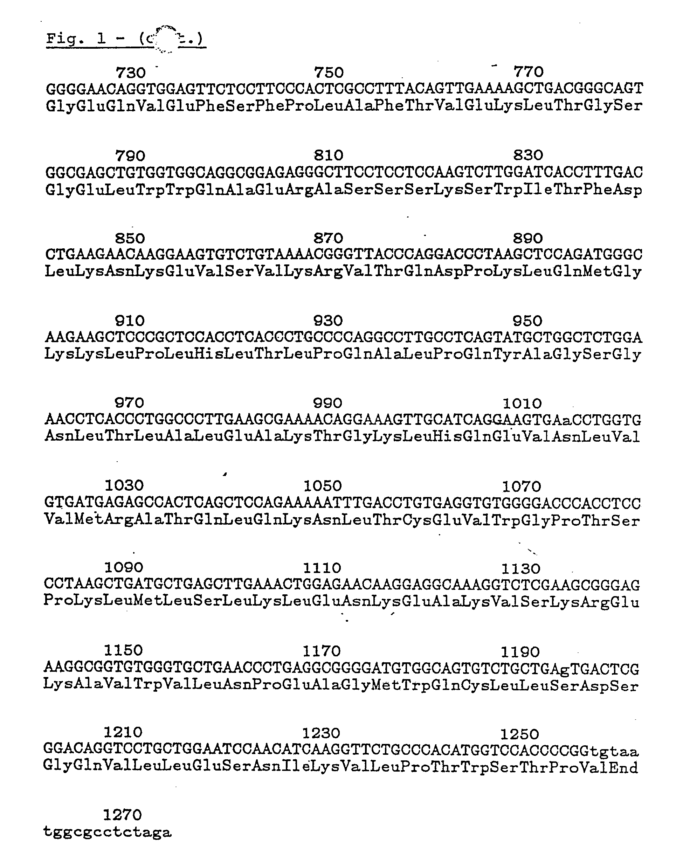

### Brief Description of Figure 1

Sequence of Soluble T4: The cDNA sequence of soluble T4 is derived from the published sequence of the T4 cDNA (Maddon, et al., Cell 42:93(1985)) and a synthetic linker added at the HpaII site at bp 1252. The synthetic linker introduced a translation termination codon at bp 1258. The predicted protein sequence is shown below the nucleotide sequence. The leader processing site was postulated to lie between the (bp 152) and gln (bp 155). The initial 20 residues of the sT4 protein, as determined by amino acid sequencing, are shown below the predicted, protein sequence; identity with the predicted sequence is indicated by an *.

### Detailed Description of the Invention

The present invention describes a means to produce a recombinant T4 molecule, designated soluble T4 (sT4), consisting of the predicted extracellular domain of the T4 receptor. (See Figure 1.) Using that portion of the T4 cDNA which encodes the leader and extracellular domains of the T4 receptor, i.e., pre-sT4, vectors are constructed capable of overexpression of sT4 in mammalian cells.

The coding sequence for sT4 can be obtained, for example, by synthesizing the gene using the known DNA sequence, by standard cloning techniques based on the sequence and by reisolation by detection of protein, i.e., transfection of cDNA clones from T4 expressing cell lines and identification by antibodies directed against the protein (See Maddon, et al., supra). cDNA clones carrying the sT4 coding sequence can be identified by use of oligonucleotide hybridization probes. The probes can be designed based on the known sequence of the T4 protein. Having identified a clone carrying the sT4 coding sequence, the coding sequence can be excised by the use of restriction endonucleases and inserted into cloning and/or expression vectors. In an expression vector, the sT4 coding sequence is operatively linked to regulatory functions required or desirable, for transcription, translation and processing of the coding sequence.

Regulatory functions include, for example, functions required for RNA polymerase binding and transcription, as well as other functions such as polyadenylation and enhancement of transcriptional sequences. The promoter can be regulatable so that, for example, expression is not induced until after transfection and selection of transformed clones. Promoters useful in the practice of the invention include, for example, the SV40 early promoter, and the long terminal repeats (LTR's) of rous sarcoma virus or moloney sarcoma virus.

Prior to transfection, the sT4 minigene, i.e., the gene encoding the leader and extracellular domains of the T4 receptor, preferably is incorporated into a larger DNA molecule which comprises a genetic selection marker system. The selection marker system can consist of any gene or genes which cause a readily detectable phenotypic change in a transfected host cell. Such phenotypic change can be, for example, foci formation, drug resistance, such as genes for G418 or hygromycin B resistance; or other selectable markers such as xanthine guanine phosphoribosyl transferase (xgprt), thymidine kinase (TX) and galactokinase (galK). A selection marker which permits gene amplification can be employed to increase copy number, whether by increased transfection efficiency or by enhanced intra-cellular replication of the gene of interest and the selection marker. Such markers which also serve to amplify gene copy number include genes for dihydrofolate reductase (methotrexate resistance), CAD (N-phosphonacetyl-L-aspartate resistance) and adenosine deaminase (2-deoxycoformycin resistance)

Following transcription and translation in mammalian cells, the leader sequence appears to be cleaved and mature sT4 is secreted into the conditioned medium.

In the preferred practice of the invention, the sT4 minigene is linked with a human H-ras or a mouse dihydrofolate reductase (DHFR) minigene to create the expression vectors.

The sT4 minigene is linked, for example, with the human H-ras or mouse DHFR in order to provide a selection marker and means to selectively amplify gene expression through co-transfection with these genes. Common selection markers include, for example, DHFR, G418 or hygromycin which select for integration of as few as a single copy of the gene of interest. Amplification with, for example, methotrexate (mtx) in the DHFR system results in overexpression of the gene.

An alternate means of increased expression of the gene includes use of the ras proto-oncogene. The ras gene family includes the H-ras, K-ras and N-ras genes. In a preferred application of the invention, the H-ras gene is used.

Other DNA functions can be linked directly or indirectly to the sT4 minigene or such functions may be unlinked. See, for example, Axel, U.S. Patent 4,399,216.

Overexpression of gene products in mammalian, cells can be achieved by transient or stable means. Transient overexpression can be achieved by viral methods such as the use of vaccinia virus vectors or by gene amplification methods such as with SV-40 based vectors in cells which support SV-40 replication. These methods ultimately lead to cell death. Stable overexpression can be achieved by generation of multiple gene copies such as through selection for gene amplification or through the use of the ras proto-oncogenes.

Overexpression of sT4 protein by co-transfection using the H-ras gene system can be achieved using a number of different cell lines, with the preferred cell line being the NIH-3T3 cell line which is a contact inhibited mouse fibroblast cell line. Other cell lines include the normal rat kidney (NRX)(ATCC 1571) cell line and the rat embryo fibroblast 52 (REF-52) cell line (McClure, et al., Cold Spring Harbor Conferences on Cell Proliferation 9:345(1982)).

When using the selection marker system, for example, DHFR with methotrexate (DHFR/MTX technique), wherein amplification of gene copy number is achieved by selective amplification, the Chinese hamster ovary cell line (CHO) is preferred. In particular CHO cells deficient in DHFR are used (Urlaub, et al., Cell 33:405 (1983)). Other cell types which may be used include, for example, any mammalian cell which has been modified so as to be DHFR⁻.

Some DHFR⁺ cell types may be used in combination with mutant DHFR genes which are less sensitive to methotrexate than normal DHFR (Axel, U.S. Patent 4,399,216). In principle, DHFR⁺ cells may be used in combination with normal DHFR genes and an additional dominant selectable gene such as the gene for G418 resistance (Kim et al, Cell 42: 129 (1987)). Transfection is carried out using standard techniques. See, for example, Wigler, et al., Proc. Natl. Acad. Sci. USA 76:1373(1979) and Copeland, et al., Cell 17:993(1979). These techniques include, for example, calcium phosphate precipitation, DEAE-dextran induced pinocytosis, electroporation and viral transfection.

Following transfection, cells harboring and expressing the resistance or marker gene are obtained by culturing in standard mammalian culture media containing a selective drug. For example, selection for DHFR resistance in DHFR deficient cells can be carried out in Ham's F12 medium without hypoxanthine and thymidine (Gibco, Grand Island, New York) containing 5-15% dialyzed fetal bovine serum; selection for DHFR resistance in DHFR⁻ cells can be carried out in Delbecco's modified Eagle medium (DMEM) (Gibco) containing 5-15% calf serum and methotrexate; selection for morphologic transformation with an H-ras gene can be carried out in DMEM in 5-10% calf serum. cells carrying a resistance gene which can amplify in response to drug concentration may be further cultured in medium containing increasing amounts of drug. Cell cultures are maintained at ambient pressure at 30 to 45° C. The selected cells which express high levels of the sT4 protein are identified by standard immunological techniques such as ELISA and immunoblotting. Such cells are cultured and the product, the sT4 protein, is collected and purified.

Conditioned medium (CM) can be collected from cells adhered to solid supports or in suspension. For example, CM is prepared from, adherent cells grown in roller bottles or grown on solid supports and cultured in suspension or in fluidized or packed beds. CM is prepared from suspension cells in stirred-tank vessels.

The sT4 of the invention includes derivatives of the extracellular domain of T4. Such derivatives comprise addition, deletions or substitutions which alterations do not significantly adversely affect secretion of the protein into the conditioned medium and the affinity of the protein for HIV env protein, i.e., gp120. For example, one or a few amino acids can be added to or deleted from the N- or C- terminus. Or, one or a few amino acids, preferably no more than four amino acids can be inserted into, deleted or substituted for internal amino acids. Alternatively, a hybrid protein, i.e., a translational fusion, can be constructed between sT4 and a protein carrier, another antigen or other sT4 molecules to prepare a poly-sT4 molecule. In yet another alternative, sT4 can be synthetically conjugated to a carrier molecule.

One embodiment of a sT4 derivative is illustrated in the Examples, below. See Example 4. Affinity of the sT4 for HIV env can be demonstrated by a competitive binding assay using a sT4 molecule having a known affinity or using antibodies which recognize the T4 receptor, such as OKT4 and OKT4A. Useful derivative sT4 molecules of the invention are selectively precipitated from conditioned medium by OKT4A as shown in Examples 4B and 4C. Derivatives can be prepared chemically, after expression, or genetically, prior to expression, by manipulation of the coding sequence for the leader and/or extracellular domain.

The sT4 of the invention can be purified from the spent culture media using various protein purification techniques, for example, affinity chromatography; ion exchange chromatography; size exclusion chromatography; hydrophobic chromatography or reversed phase chromatography.

sT4 can be purified by affinity chromatography using general group-specific adsorbants, for example, carbohydrate binding or dye affinity ligands; or using ligands that specifically bind to sT4, for example, monoclonal antibody or HIV gp120 protein or portions thereof.

An exemplary purification scheme comprises: 1) growing cells in a serum-free selection growth media, 2) clarification of the conditioned media, and 3) separation of sT4 of the invention from other proteins present in the conditioned media.

In the preferred method, the sT4 is purified from the serum-free culture medium using a series of chromatography steps which are based on the physical properties of the sT4 molecule. The sT4 may also be purified from culture medium containing serum using similar chromatography methods.

In the preferred method of purification of sT4, culture medium is first clarified, such as by centrifugation and/or filtration and is then passed through a cation exchange support, e.g., a carboxymethyl or sulfopropyl column, preferably an S-Sepharose® (Sulfopropyl Sepharose) (Pharmacia, Piscataway, New Jersey) column, which binds the sT4 while the majority of contaminating proteins flow-through the column. The protein sample is then eluted using a linear salt gradient, e.g., 0-0.5 M NaCl. A second ion exchange column is used preferably following dialysis to remove salt. This column is an anion exchange column, e.g., a diethylaminoethyl or quaternary aminoethyl column, preferably a Q-Sepharose® (quarternary amino ethyl Sepharose) (Pharmacia) column, has properties such that the contaminating proteins present in the sample are bound to the column while the sT4 does not bind and is recovered in the column flow-through buffer. Finally, a gel filtration column, such as, a cross-linked dextran column or an agarose column, is used which acts to remove remaining contaminating materials.

The size exclusion step is preferably carried out using a small particle packing, e.g., less than about 50 um, having a separation range of 1000 to 300,000 molecular weight. It has been discovered that by use of such gel, residual traces of high molecular weight contaminants can be removed. An example is Superose® 12 cross-linked agarose (Pharmacia) which has an average particle size of 20-40 um and a separation range of 1,000 to 300,000 molecular weight.

An alternative method of purification of sT4 involves the use of monoclonal antibodies directed against sT4. The sT4 protein can be purified in one step by passage of clarified culture media through an affinity gel support to which monoclonal antibody directed against sT4 is bound. The sT4 will bind to the column at the antibody binding site while all contaminating proteins wash through the column.

The sT4 is then eluted from the column under conditions that prevent sT4 protein from being inactivated.

Characterization of the in vitro biological and immunological properties of the sT4 protein indicate the protein is of value in the prevention and treatment of AIDS. Studies indicate the sT4 protein acts as an inhibitor of extracellular and cell to cell spread of the virus. Because sT4 has been shown to block virus binding to T4[+] target cells in culture, it is believed administration of sT4 to infected persons would act to inhibit the extracellular spread of the virus. Therefore, sT4 is of value both as a prophylactic and therapeutic agent for treatment of AIDS.

As a prophylactic, sT4 is administered to individuals at high-risk for the disease or individuals who show exposure to HIV by the presence of antibodies to virus. Administration of an effective amount of sT4 at an early stage of the disease or prior to its onset acts to inhibit infection of T4[+] lymphocytes by HIV. As a therapeutic, administration of sT4 to persons infected with HIV acts to inhibit extracellular spread of the virus.

Fusion between HIV infected cells and other T4[+] lymphocytes also appears to be a route of virus spread. Further, fusion may be responsible, in part, for the impairment of T4[+] lymphocyte function and ultimately the depletion of T4[+] lymphocytes in infected individuals. Cell fusion is dependent on both viral envelope gene products and the T4 receptor and can be inhibited by the OKT4A, or similar monoclonal antibodies (Mabs) (Sattentau, et al., Science 234:1120(1986)). sT4 interferes with cell fusion and therefore is expected to diminish the cell to cell spread of virus and the loss of T4[+] lymphocyte function.

The T4 receptor is monomorphic and thus sT4 is believed to be a universal inhibitor of virus which recognize the surface domain of the T4 receptor, including all HIV.

sT4 may be used in combination with other agents, for example, in association with agents directed against other HIV proteins, such as reverse transcriptase, protease or tat. An effective therapeutic agent against HIV should prevent virus mediated as well as cell to cell transmission of infection. sT4 may also be used in combination with other anti-viral agents, for example, azidothymidine (AZT).

The sT4 protein of this invention also has utility as an inhibitor of T4[+] cell function. Numerous studies suggest a critical role for the CD4 receptor (CD4 is general terminology for the human T4 receptor and its counterparts in other mammalian cells) in immune tolerance, particularly in the pathogenesis and progression of autoimmune diseases, and in host specific graft rejection. Of particular relevance to sT4 are the observations with anti-CD4 Mabs. Through their association with the CD4 receptor, certain of these Mabs ameliorate autoimmune responses and graft rejection. Examples of such action include inhibition of T-cell function in vitro, for example, antigen induced proliferation, lymphokine secretion and helper cell function by certain anti-CD4 Mabs; treatment of systemic lupus erythematosus by administration of anti-CD4 Mabs to retard the onset of murine lupus; and grafting studies in mice wherein a single dose of murine Mab directed against the murine CD4 receptor results in acceptance of the allograft.

The molecular consequence of the binding of Mab to CD4 is unclear. The Mabs may block the association of CD4 with its ligand, which ligand, evidence suggests, is a conserved epitope on MHC class II antigens (see Greenstein et al., Ann. Inst. Pasteur 138:134(1987) and Gay, et al., Ann. Inst. Pasteur 138:127(1987)). However, at least some of these same Mabs inhibit CD4 cell activation by an apparent class II independent path.

sT4 is also believed to inhibit T cell interactions as a competitor of cellular T4, perhaps by binding to extracellular target molecules which normally interact with the surface domain of the T4 receptor. This distinction between Mabs and sT4 could have important functional consequences. For example, whereas some Mabs directed against T4 elicit a response on the T4 cell, sT4 may elicit a response on cells expressing MHC class II antigens. Also the affinity of T4 for its presumed class II ligand appears to be quite low compared to the high affinities of Mabs directed against T4. Thus, although Mabs and sT4 may interfere with the same processes, they would affect different target molecules and different target cells.

As a prophylactic or therapeutic, sT4 is administered parenterally, preferably intravenously. The agent can be administered by infusion or by injection, e.g., daily, weekly or monthly. The amount and rate of sT4 administration is selected so as to maintain an effective amount of sT4 in circulation. An alternative mode of administration would be extracorporal, employing sT4 as a dialysis agent.

The sT4 protein of this invention can also be used as a reagent to identify natural, synthetic or recombinant molecules which act as therapeutic agents or inhibitors of T4+ cell interactions.

For example, sT4 protein can be used in screening assays, such as, assays for protein interaction measured by ELISA based methodologies, to provide a biochemically pure, aqueous soluble reagent which may be used in combination with other reagents to assay for competitors of the T4 receptor surface domain interactions. For example, since sT4 binds to HIV env protein or mixtures containing HIV env proteins, it can be used to screen for inhibitors of virus binding. Based on in vitro data showing that sT4 binds to cells expressing HIV env proteins, sT4 can also serve as a selective targeting molecule for HIV infected cells in vivo. As a target specific carrier proteins sT4 can serve, for example, as the carrier protein for delivery of cytotoxic agents to the infected cells.

In addition, based on data showing that the T4 receptor specifically associates with MHC class II antigens on antigen presenting cells as suggested by the class restriction of T4+ cells, sT4 can be used in combination with class II antigens to test for inhibitors of T4 lymphocyte - target cell interactions. In addition to these examples, which are based on direct binding assays between sT4 and its target molecules, more complex assays can be designed which rely on the biochemical responses to sT4 recognition.

The sT4 can be used in diagnostic assays for the detection of T4 proteins or the molecules with which they interact. For example, quantitation of T4 and T4+ cells and antibodies to T4 would be of diagnostic value for AIDS.

In addition, sT4 can be used to generate new diagnostic reagents, for example, Mabs or other types of molecules for use in the standard immunologic assays, ie., ELISA, capture immunoassays, radioimmune assays. Because sT4 displays the OKT4, the OKT4A and most if not all of the other surface epitopes of the T4 receptor, sT4 is especially useful in immunodiagnostic assays as it can be used for absolute quantitation of T4 levels in a system. Currently there are no standards for quantitating the T4 receptor.

The T4 receptor resides in three diverse chemical environments: the oxidizing, hydrophylic cell surface; the hydrophobic membrane; and the reducing, hydrophylic cytoplasm. These diverse environments would most likely preclude the isolation of the receptor in its fully native state. sT4, which consists of only the extracellular domain, is secreted as a soluble protein into the cell supernatant and its conformation appears to mimic the surface of the receptor surface domain. Thus, sT4 is suitable for detailed structural analysis, in particular for x-ray crystallography. Determination of the 3-dimensional structure of sT4 alone or in a complexed form with other interactive molecules could provide a basis for the rational design of selective antagonists and agonists for sT4.

## Examples

The examples which follow are illustrative, and not limiting of the invention. Enzymes used in the genetic manipulations were obtained from commercial sources and were used substantially in accordance with the vendor's instructions. Except where otherwise indicated, procedures were carried out substantially as described by Maniatis, et al., Molecular Cloning, Cold Spring Harbor Laboratory, 1982.

### Example 1: Construction of vectors

Using recombinant DNA manipulations, base pairs (bp) 1-1257 of the human T4 cDNA sequence (Maddon, et al., supra) were placed between an SV-40 early promoter and a TAA termination codon followed by the polyadenylation region of the bovine growth hormone gene. This sequence of the T4 cDNA encodes the leader and predicted extracellular domain of the T4 receptor. This sT4 minigene was joined with a human H-ras, or a mouse dihydrofolate reductase, minigene to create the vectors PsT4CHraS and PsT4DHFR respectively. The construction of these vectors was as follows:

## Construction of pST4sal:

Plasmid pST4sal was constructed from two other plasmids, JRT4 and PUCsT4. The construction of these plasmids is detailed below.

## Construction of plasmid JRT4:

To create plasmid JRT4, plasmid DSPI (Pfarr, et al., DNA 4:461(1985)) was cut with Xhol, the SV40 polyA early region was deleted, and the Xhol sites were filled in using Klenow fragment of DNA polymerase. The bovine growth hormone (BGH) polyadenylation region (Pfarr, et al., DNA 5:115(1986)) was cut with Pvull and Kpnl, and the Kpnl site was blunted by treatment with T4 DNA polymerase. This 230 bp fragment was ligated to DSPI to create DSPIBGH.

DSPIBGH was cut with Smal and Sall and the galK cassette (consisting of the SV40 early promoter, galK coding region and BGH polyA region) was ligated into pUCl9 (Yanisch-Perron, et al., Gene 33:103(1985)) at the Sall site by using a synthetic linker consisting of a Sall end, a Bglll site, and a Smal end. This three part ligation resulted in plasmid DSPIBZBGH.JT.

DSPIBZBGH.JT, which was cut with Stul and Bcll to delete the galK coding region, was ligated to a 1.7 kb EcoRI (filled in)-BamHI fragment, containing the T4 cDNA from plasmid pT4B (Maddon, et al., Cell 42:93(1985)) to create plasmid JRT4.

## Construction of plasmid PUCsT4:

To create plasmid PUCsT4, a Haell and Hpall fragment (1125 bp) of the T4 cDNA from plasmid pT4b was ligated through the use of synthetic linkers, to vector pUC18 which had been cut with Kpnl and Xbal. The Haell end of the T4 cDNA was ligated to the Kpnl site of pUC18 using a synthetic linker with a Kpnl end and a Haell end. The Hpall end of the T4 cDNA was ligated to the Xbal site of pUC18 using a synthetic linker with a Hpall end and an Xbal end. This linker also inserted a TAA stop codon after nucleotide 1257 of the T4 coding region. The resulting plasmid was PUCsT4.

To create plasmid psT4sal, plasmid JRT4 was cut with Bglll and Sacl and a 959 bp fragment (consisting of the SV40 early promoter and the first 602 nucleotides of the T4 cDNA) was isolated. Plasmid PUCsT4 was cut with Sacl and Xbal and a 660 bp fragment (consisting of the T4 cDNA from nucleotides 603-1257 followed by the TAA codon from the synthetic linker) was isolated. These two fragments were ligated into DSPIBZBGH.JT which had been cut with Bglll and Xbal to delete the SV40 early promoter and full length T4 coding region. The resulting plasmid was pST4sal.

## Construction of pST4DHFR:

To create plasmid pST4DHFR, a Bglll-BamHI fragment containing a β-globin DHFR expression cassette was ligated into the BamHI site of pST4sal. The β-globin DHFR expression cassette consists of: the mouse β-globin promoter (550 bp Hincll fragment from plasmid pPK288 (Berg, et al., Mol. cell. Biol. 3:1246(1983)) modified at its 5' end with a synthetic linker to contain a Bglll site; the mouse

DHFR coding region (735 bp HindIII (fill-in) fragment from plasmid pSV2-DHFR (Subramani, et al., Mol. cell. Biol. 1:854(1981)); Nhel (fill-in)-BamHI (fill-in) SV40 polyA early region from DSPI (Pfarr, et al., DNA 4:461(1985)); and the mouse DHFR terminator region (907 bp HindIII (fill-in fragment from plasmid mDH9 (Frayne, et al., Mol. cell. Biol. 4:2921(1984)), modified at its 3' end with a synthetic linker to create a BamHI site.

## Construction of pST4cHras:

Plasmid pmERcHras was created by cutting plasmid pMER with EcoRV and HindIII (fill-in), to remove the galK region, and ligating in an 870 bp Ndel (fill-in)-Sall (blunted by mung bean nuclease) fragment, containing the coding region for cHras, from plasmid psKcHras (Gross, et al., Mol. cell. Biol. 5:1015(1985). Plasmid pMER differs from pSVK (Schumperli, et al., Proc. Natl. Acad. Sci. USA 79:257(1982)) at four positions: (1) deletion of the HindIII site at bp 340 by insertion of a linker containing a Smal site; (2) insertion of a 34 bp EcoRI to HindIII fragment from pUC9 (Vieira, et al., Gene 19:259(1982)) into the Pvull site at bp 1; (3) deletion of a 722 bp Hpal fragment at bp 1573-2295; and (4) deletion of the Apal site at bp 2701 by insertion of a linker containing a Sall site.

The soluble T4 transcription cassette was removed from pST4sal via a Bglll-BamHI fragment and ligated into the BamHI site (3' to the SV40 polyA early) of pMERcHras to create pST4cHras.

## Example 2: Expression of soluble T4 (sT4) minigenes in mammalian cells

### A. Expression of pST4cHras in NIH-3T3 cells:

Plasmid pST4cHras (10 μg) was co-precipitated, by the calcium phosphate precipitation method, with 10 μg of plasmid pTKneo, a vector conferring G418 resistance, in the presence of 10 μg carrier DNA (NIH-3T3 genomic DNA), onto NIH-3T3 cells seeded at $5 \times 10^5$ cells per 60 mm culture dish the preceding day. The cells were incubated with the precipitated DNA for 6 hours at 37° C. The DNA precipitate was removed and fresh media (DMEM, 5% Nu-Serum® (Collaborative Research, Inc., Lexington, Massachusetts)) was added to the dishes. The cells were trypsinized 16 hours later and seeded into three 100 mm dishes and maintained in the above media. Foci (approximately 50 per dish) appeared in 12-14 days. Eleven of the transformed foci were selected, expanded and then seeded at $5 \times 10^5$ cells per 100 mm dish for selection in the above media plus 500 μg/ml GENETICIN® G418 (Gibco Laboratories, Grand Island, New York). All 11 clones survived G418 selection (500 μg/ml) and were screened for H-ras (p21) levels by standard protein immunoblot analysis.

The clones which expressed the highest levels of p21 (approximately 2 ng p21/μg Triton-soluble protein) were assayed for expression of sT4. Confluent cultures were incubated for 18 hours with 35S-labelled methionine and cysteine. Culture supernatants and cell lysates were immunoprecipitated with monoclonal antibodies specific for the T4 (OKT4, OKT4A) and the T8 (OKT8) receptors, polyclonal antibody specific for ras proteins, or nonspecific mouse IgG. A protein of about 45 kd, the predicted size of sT4, was specifically precipitated from the culture medium by both of the monoclonal antibodies directed against the T4 receptor. The sT4 band was not observed in cell lysates. As expected, p21 was precipitated from the cell but not from the culture supernatants. Subsequent quantitation shows, compared to purified sT4, these cells produce relatively low levels of sT4, i.e., approximately 100 fold lower than with CHO cells as described in Example 2B.

B. Expression of pST4DHFR in Chinese Hamster Ovary (CHO) cells:

DXB-11 cells, a DHFR deficient CHO cell line (Urlaub, et al., supra) were transfected by calcium phosphate precipitation with 10 to 30 μg of pST4DHFR in the presence of 10 μg carrier DNA (NIH-3T3 genomic DNA), one day after seeding 60 mm dishes with $5 \times 10^5$ cells. cells were incubated with the DNA precipitate for 6 hours at 37° C, the media was removed, and fresh media (F12, 10% FBS, 100 units/ml penicillin and streptomycin) was added to the dishes. The media was changed again after 16 hours and the cells were incubated for another 24 hours. The cells were then trypsinized, seeded into three 100 mm dishes and selected in nucleoside free media (F-12 without hypoxanthine and thymidine, 10% dialyzed FBS, and 100 units/ml penicillin and streptomycin). Colonies (approximately 100 per dish) appeared in 7-10 days. Colonies from each dish were pooled, expanded and then seeded at $5 \times 10^3$ and at $5 \times 10^4$ cells per well in 24 well culture plates, or at $5 \times 10^5$ cells per 100 mm dish. The cells were allowed to recover for 3 days before beginning selection in nucleoside free media containing 80 nM methotrexate (mtx). Individual wells or clones were assayed at confluence for sT4 expression, and those selected for further amplification were seeded into 24 well culture plates at the densities described above. Selection at 800 nM mtx in nucleoside free media was started 3 days after seeding. This selection procedure was repeated for selections at 8 μM and 80 μM mtx.

Several cell lines were derived using this method which express soluble T4 at a minimum of 3 pg/cell/24 hrs.

Example 3: Purification of sT4

Conditioned medium (CM) was prepared serum-free from adherent cell cultures expanded into 850 cm² roller bottles under mtx selective conditions. At confluence, the cells were washed twice with phosphate buffered saline (PBS) without $Mg^{2+}$ and $Ca^{2+}$ and the growth medium (Ham's F12 without hypoxanthine and thymidine, 10% fetal bovine serum, 100 units/ml penicillin and streptomycin and mtx at the selective concentration) was replaced with the same medium minus serum and mtx and plus 1 x ITS (insulin, transferrin and selenium (Collaborative Research Inc.). After 24-48 hrs. the medium was removed and replaced with selective growth medium. Serum-free medium was then reapplied within 3-5 days and this cycle repeated indefinitely, i.e., for more than two months. CM was clarified by centrifugation at 8,000 x g. A protease inhibitor PMSF (phenylmethylsulfonylfluoride) was added to 0.5 mM and the CM was concentrated about 10x fold by pressure membrane filtration. This concentrated CM was clarified by centrifugation at 2000 x g and Aprotinin, protease inhibitor, (Sigma Chemical, St. Louis, Missouri) was added to a final concentration of 5 μg/ml. The sample was processed directly or after storage at -70° C.

The concentrated CM sample was diluted 2-fold with 50 mM MES [2-(N-morpholino)-ethanesulfonic acid], pH 6.0 and filtered through a 0.45 micron filter. The sample was then treated with 100 μM pAPMSF (p-amidinophenylmethylsulfonylfluoride) (Cal-Biochem-Behring, San Diego, California) and applied to a S-Sepharose® (Sulpho-propyl) (Pharmacia P-L Biochemicals, Piscataway, New Jersey) column equilibrated in 50 mM MES, pH 6.0 at a protein concentration of 1.5 - 2.0 mg/ml gel. The sample was eluted using a linear gradient of 0 - 0.5 M NaCl in 50 mM MES, pH 6.0. Peak fractions which eluted at approximately 0.2 M NaCl were pooled and treated with PAPMSF to 100 μM. Fractions containing sT4 were confirmed by SDS-PAGE and immunoblot assays. After sitting at 4° C for 1 hour the sample was dialyzed against 50 mM Bis-Tris propane [1,3-bis[tris-(hydroxymethyl)methyl amino]propane], pH 6.0.

The sample was treated with 100 μM pAPMSF and 0.1% thiodiglycol, pH 9.0 was then applied to a Q-Sepharose® (quarternary amino ethyl) column (Pharmacia) (5 ml sample/ml gel) equilibrated in 50 mM Bis-Tris propane (BTP), pH 9.0. The sT4 sample does not bind to the Q-Sepharose and was recovered in the unbound fraction and column wash. The unbound sample was immediately adjusted to pH 6.0.

The final step was chromatography on a 30 micron Superose® 12 column (2.5 x 46 cm) (Pharmacia) equilibrated in 50 mM phosphate, 0.15 M NaCl pH 7.0. The column was run at a flow rate of 3.0 ml/min. Ten ml injections were made and the 42 minute peak was collected batchwise. The process yielded approximately 1.0 mg of product per 20.0 mg of total protein for a cell line producing approximately 3 pg/cell/day.

Example 4: Characterization of the T4s

A. Physical properties

Total protein concentration was determined using the colormetric BCA protein assay (Bicinchoninic Acid, Pierce Chemical Co., Rockford, Ill.). Absolute concentrations were determined by quantitative amino acid analysis. The measured amino acid composition of the purified sT4 was performed using standard amino acid analysis techniques and was found to agree with the predicted sequence for the molecule to within experimental error (+/- 15%). Through the first 20 residues the sequence was as predicted except that it begins lys-lys-val-val----. Thus, the mature amino terminus begins at position +3 with respect to the predicted leader clip site (Maddon, et al., supra., (1985)) and differs from the predicted sequence at that position by a asn to lys change. The position of the mature amino terminus agrees well with the determined termini of the mouse and sheep CD4 proteins. It is speculated that the asn to lys change may represent a sequencing error (single base change) or a mutation which arose during recombinant procedures.

B. Immuno-epitopes:

The monoclonal antibodies OKT4 and OKT4A recognize non-interfering surface epitopes of the T4 receptor (Rao, et al., Cell Immunol. 80:310(1983)). These antibodies are specific for the native conformation in that they will not bind to reduced, SDS-denatured protein in immuno-blot assays. It was shown that both antibodies specifically precipitate sT4 from $^{35}$S-labelled culture supernatants using the following immunoprecipitation procedure:

Cultures of sT4 producing cells containing 1 X 10$^6$ cells per 60 mm culture dish, were labelled for 16 hours at 37° C in 1.5 ml methionine and cysteine free F12 medium containing ITS, and 170 µCi/ml [$^{35}$S]methionine and 30 µCi/ml [$^{35}$S]cysteine (ICN Biomedicals, Inc., Costa Mesa, CA). Clarified medium (100 µl) was diluted with an equal volume of precipitation buffer (10mM sodium phosphate pH 7.5, 100 mM NaCl, 0.1% NP-40®, 0.5% non-fat dry milk) and incubated with 3 µg rabbit IgG for 15 min. at 4° C followed by 30 µl (packed volume) of protein A sepharose beads (Pharmacia P-L Biochemicals) for 30 min. at 4° C. The precleared supernatant was incubated with 5 µg of OKT4, OKT4A and OKT8 (gift of P. Rao, Ortho Pharmaceuticals Corp., Raritan, New Jersey), mouse IgG (Cooper Biomedical, Malvern, PA), or rabbit α-mouse IgG (Cooper Biomedical) for 30 min. at 4° C. OKT4, OKT4A, OKT8, mouse IgG, and rabbit α-mouse IgG were precipitated by incubation with 20 µl (packed volume) of protein A sepharose beads for 30 min. at 4° C. Following precipitation, the beads were washed twice with 200 µl precipitation buffer and once with 200 µl precipitation buffer minus NP-40® and non-fat dry milk. The washed beads were boiled for 5 min. in 20 µl sample buffer (125 mM Tris-HCl pH 6.8, 20% glycerol, 1.4 M β-mercaptoethanol), and the supernatants were analyzed by electrophoresis on a 12.5% SDS-polyacrylamide gel. Similar results were obtained with OKT4B, 0KT4C, OKT4D, OKT4E, 0KT4F and other Mabs specific for T4. These results suggest that the conformation of sT4 accurately mimics the surface domain of the T4 receptor.

C. Biochemical and biological properties

1. Recognition of gpl20 - Binding of sT4 to gpl20 was assessed essentially as described for measuring the interaction between gpl20 and the full-length T4 receptor (McDougal, et al., supra., (1986)). In this assay, a lysate of extracellular HIV (LAV I strain) from infected cells labelled with $^{35}$S-methionine and $^{35}$S-cysteine is incubated with a T4 protein sample. The T4-gpl20 complex is immunoprecipitated with OKT4 antibody which does not interfere with the binding of gpl20 to T4. In initial assays, CM from sT4 producing cells was compared with similarly prepared CM from control DXB-11 cells. HIV gpl20 was precipitated when incubated with CM from the sT4 producing cells but not with CM from the DXB-11 cells. In subsequent assays, purified sT4 specifically co-precipitated gpl20.

2. Competition of HIV binding - The binding of gpl20 to sT4 suggested that sT4 would inhibit the binding of the AIDS virus to susceptible cells. CM containing or lacking sT4 as described in C.1., above, was used in the initial assays. HIV was incubated with the CM and virus binding to the T4$^+$ CEM cell line was quantitated by incubation with a FITC conjugated, anti-HIV antibody and FACS (fluorescein activated cell sorter) analysis as described by McDougal, et al., supra., (1985). CM from the sT4 producing cell line inhibited HIV binding in a dilution dependent manner, whereas no response was seen with CM from matched non-producer (DXB-11) cells. In subsequent assays, preincubation of virus with purified sT4 at 8.2 µg/ml completely blocked virus binding.

3. Competition of HIV infection - Inhibition of HIV infection was assessed according to methods described by McDougal, et al., supra (1985). In initial assays, HIV infectivity titrations were carried out in the presence of concentrated CM from cells expressing sT4 at approximately 0.15 pg/cell/day. Serial dilutions of virus were incubated in 6x concentrated CM and then added to phytohemagglutinin stimulated normal lymphocytes. After an overnight incubation, the cells were washed and then maintained in medium containing 2x CM. The presence of virus was assayed at days 4, 8 and 12 by an antigen capture assay. CM from the sT4 producing cells inhibited virus infectivity 90% relative to control CM or no addition. Using purified sT4 at a concentration of 8.6 µg/ml, a 4-log inhibition of infection was observed at day 8 after infection with a 10- infectious dose of virus.

The above description and examples fully disclose the invention including preferred embodiments thereof.

Modifications of the methods described that are obvious to those of ordinary skill in molecular genetics and related 6ciences are intended to be within the scope of the following claims.

**Claims**

1. A process for purifying sT4 from clarified conditioned medium from a cell culture which produces sT4 which comprises (i) contacting the conditioned medium with a cation exchange support whereby the sT4 is adsorbed to the support, (ii) eluting the sT4 from the support, (iii) contacting the eluate with an anion exchange support and (iv) collecting sT4 in the effluent from the anion exchange support.

2. The process of claim 1 wherein the cation exchange support is a carboxymethyl or sulfopropyl support; the sT4 is eluted with an increasing salt gradient; and the anion exchange support is DEAE or QAE.

3. The process of claim 1 which further comprises passing the effluent from the anion exchange support through a size exclusion gel.

4. The process of claim 4 wherein the size exclusion gel is cross-linked agarose having a particle size of less than 50 um and having a separation range of 1000 to 300,000 molecular weight.

5. A process for producing sT4 which comprises (i) culturing mammalian cells which express sT4; (ii) clarifying the conditioned medium by centrifugation and filtration; (iii) contacting the clarified medium with a sulfopropyl-Sepharose® column; (iv) eluting sT4 from the column with a gradient of 0 - 0.5 M NaCl; (v) dialyzing the eluate to remove the salt; (vi) contacting the dialyzed eluate with a QAE-Sepharose® column; (vii) passing the effluent through a column of Superose® 12 and (viii) collecting the purified sT4.

## Figure 1

```
        10                30                50
CAAGCCCAGAGCCCTGCCATTTCTGTGGGCTCAGGTCCCTACTGCTCAGCCCCTTCCTCC


        70                90                110
CTCGGCAAGGCCACAATGAACCGGGGGAGTCCCTTTTAGGCACTTGCTTCTGGTGCTGCAA
                    MetAsnArgGlyValProPheArgHisLeuLeuLeuValLeuGln


        130               150               170
CTGGCGCTCCTCCCAGCAGCCACTCAGGGAAACAAAGTGGTGCtGGGCAAAAAAGGGGAT
LeuAlaLeuLeuProAlaAlaThrGlnGlyAsnLysValValLeuGlyLysLysGlyAsp
                          Lys  *   *   *   *   *   *   *   *   *


        190               210               230
ACAGTGGAACTGACCTGTACAGCTTCCCAGAAGAAGAGCATACAATTCCACTGGAAAAAC
ThrValGluLeuThrCysThrAlaSerGlnLysLysSerIleGlnPheHisTrpLysAsn
 *   *   *   *   *   *   *   *   *   *


        250               270               290
TCCAACCAGATAAAGATTCTGGGAAATCAGGGCTCCTTCTTAACTAAAGGTCCATCCAAG
SerAsnGlnIleLysIleLeuGlyAsnGlnGlySerPheLeuThrLysGlyProSerLys


        310       ,       330               350
CTGAATGATCGCGCTGACTCAAGAAGAAGCCTTTGGGACCAAGGAAACTTCCCCCTGATC
LeuAsnAspArgAlaAspSerArgArgSerLeuTrpAspGlnGlyAsnPheProLeuIle


        370               390               410
ATCAAGAATCTTAAGATAGAAGACTCAGATACTTACATCTGTGAAGTGGAGGACCAGAAG
IleLysAsnLeuLysIleGluAspSerAspThrTyrIleCysGluValGluAspGlnLys


        430               450               470
GAGGAGGTGCAATTGCTAGTGTTCGGATTGACTGCCAACTCTGACACCCACCTGCTTCAG
GluGluValGlnLeuLeuValPheGlyLeuThrAlaAsnSerAspThrHisLeuLeuGln


        490               510               530
GGGCAGAGCCTGACCCTGACCTTGGAGAGCCCCCCTGGTAGTAGCCCCTCAGTGCAATGT
GlyGlnSerLeuThrLeuThrLeuGluSerProProGlySerSerProSerValGlnCys


        550               570               590
AGGAGTCCAAGGGGTAAAAACATACAGGGGGGGAAGACCCTCTCCGTGTCTCAGCTGGAG
ArgSerProArgGlyLysAsnIleGlnGlyGlyLysThrLeuSerValSerGlnLeuGlu


        610               630               650
CTCCAGGATAGTGGCACCTGGACATGCACTGTCTTGCAGAACCAGAAGAAGGTGGAGTTC
LeuGlnAspSerGlyThrTrpThrCysThrValLeuGlnAsnGlnLysLysValGluPhe


        670               690               710
AAAaTAGACATCGTGGTGCTAGCTTTCCAGAAGGCCTCCAGCATAGTCTATAAGAAAGAG
LysIleAspIleValValLeuAlaPheGlnLysAlaSerSerIleValTyrLysLysGlu
```

Fig. 1 - (c    .)

```
     730               750               770
GGGGAACAGGTGGAGTTCTCCTTCCCACTCGCCTTTACAGTTGAAAAGCTGACGGGCAGT
GlyGluGlnValGluPheSerPheProLeuAlaPheThrValGluLysLeuThrGlySer


     790               810               830
GGCGAGCTGTGGTGGCAGGCGGAGAGGGCTTCCTCCTCCAAGTCTTGGATCACCTTTGAC
GlyGluLeuTrpTrpGlnAlaGluArgAlaSerSerSerLysSerTrpIleThrPheAsp


     850               870               890
CTGAAGAACAAGGAAGTGTCTGTAAAACGGGTTACCCAGGACCCTAAGCTCCAGATGGGC
LeuLysAsnLysGluValSerValLysArgValThrGlnAspProLysLeuGlnMetGly


     910               930               950
AAGAAGCTCCCGCTCCACCTCACCCTGCCCCAGGCCTTGCCTCAGTATGCTGGCTCTGGA
LysLysLeuProLeuHisLeuThrLeuProGlnAlaLeuProGlnTyrAlaGlySerGly


     970               990              1010
AACCTCACCCTGGCCCTTGAAGCGAAAACAGGAAAGTTGCATCAGGAAGTGAaCCTGGTG
AsnLeuThrLeuAlaLeuGluAlaLysThrGlyLysLeuHisGlnGluValAsnLeuVal


    1030              1050              1070
GTGATGAGAGCCACTCAGCTCCAGAAAAATTTGACCTGTGAGGTGTGGGGGACCCACCTCC
ValMetArgAlaThrGlnLeuGlnLysAsnLeuThrCysGluValTrpGlyProThrSer


    1090              1110              1130
CCTAAGCTGATGCTGAGCTTGAAACTGGAGAACAAGGAGGCAAAGGTCTCGAAGCGGGAG
ProLysLeuMetLeuSerLeuLysLeuGluAsnLysGluAlaLysValSerLysArgGlu


    1150              1170              1190
AAGGCGGTGTGGGTGCTGAACCCTGAGGCGGGGATGTGGCAGTGTCTGCTGAgTGACTCG
LysAlaValTrpValLeuAsnProGluAlaGlyMetTrpGlnCysLeuLeuSerAspSer


    1210              1230              1250
GGACAGGTCCTGCTGGAATCCAACATCAAGGTTCTGCCCACATGGTCCACCCCCGGtgtaa
GlyGlnValLeuLeuGluSerAsnIleLysValLeuProThrTrpSerThrProValEnd


    1270
tggcgcctctaga
```

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,P | WO-A-8 801 304 (THE TRUSTEES OF COLUMBIA UNIVERSITY IN THE CITY OF NEW YORK) <br> * Page 73, lines 14-21 * <br> --- | 5 | C 12 P 21/00 <br> C 07 K 3/22 // <br> C 12 N 15/00 |
| A | CELL, vol. 42, no. 1, August 1985, pages 93-104, MIT; P.J. MADDOn et al.: "The isolation and nucleotide sequence of a cDNA encoding the T cell surface protein T4: A new member of the immunoglobulin gene family" <br> * Figure 3, fragment T4B * <br> --- | 1-4 | |
| A | EP-A-0 220 966 (CETUS CORP.) <br> * Page 10, line 25 - page 11, line 58 * <br> ----- | 1-4 | |
| | | | TECHNICAL FIELDS SEARCHED (Int. Cl.4) |
| | | | C 12 P <br> C 12 N <br> C 07 K |

The present search report has been drawn up for all claims

| Place of search | Date of completion of the search | Examiner |
|---|---|---|
| THE HAGUE | 23-12-1988 | CUPIDO M. |

EPO FORM 1503 03.82 (P0401)